# EUROPEAN PATENT APPLICATION

(11) **EP 3 685 729 A1**
(43) Date of publication of application: **29.07.2020**
(21) Application number: 19153256.3
(22) Date of filing: 23.01.2019
(51) Int. Cl.: A61B 1/00, G06T 7/00, A61B 1/227, A61B 5/107

(54) **METHOD AND APPARATUS FOR OBTAINING A 3D MAP OF AN EARDRUM**

(71) Applicant: Universiteit Antwerpen, 2000 Antwerpen (BE)
(72) Inventor: Sam, Van der Jeught, 2000 Antwerpen (BE); Joris, Dirckx, 2000 Antwerpen (BE)
(74) Representative: IP HILLS NV

(57) **Abstract**

Example embodiments describe an otoscope comprising a projector for projecting a structured illumination pattern onto an eardrum; and a camera for capturing a reflection of the structured illumination pattern; and means for exchanging the reflection with the circuitry according to claim 6 for constructing a three-dimensional map of the eardrum.

## Description

### Technical Field

The present invention relates to a method and apparatus for obtaining a three-dimensional map of an eardrum.

### Background

An eardrum, also called tympanic membrane, is a thin, cone-shaped membrane separating an external ear from the corresponding middle ear. It transmits sound from the surrounding air to ossicles inside the middle ear to the oval window in the fluid-filled cochlea. The eardrum thus converts and amplifies vibrations in the air to vibrations in fluid.

The eardrum comprises a three-dimensional shape, whereby the form and condition are indicative for various diseases of the middle ear space. To properly examine a patient for illness during check-ups and diagnose ear symptoms, commonly medical doctors use theretofore an otoscope.

An otoscope is a medical device which allows doctors to look into ears. Typically, the otoscope comprises a handle, a head and a speculum for inserting in the external acoustic meatus. The head comprises a light source and a magnifying lens. By shining light onto the eardrum, the doctor investigates via the magnifying lens the condition thereof through observed reflection of the emitted light.

The eardrum is partly translucent such that the reflection comprises two components. The first component comprises light that is directly reflected from the eardrum, while the second component comprises light beams reflected from the middle ear behind the eardrum.

Because of these two components, the shape of the eardrum is indirectly observed by the doctor. Furthermore, the reflection comprising both components is represented on the magnifying lens as a two-dimensional image. This way a doctor must rely on his experience and expertise to properly make a diagnose based on the two-dimensional image.

A way to overcome the problem of the partly transparency of the eardrum is to dye the eardrum to increase the visibility for the doctor for performing a diagnosis. Drawbacks, however, are that this increases the time needed to perform the procedure and may cause undesired side effects for the patient.

Another way to increase the accuracy of diagnoses is using a method and apparatus for reconstructing a three-dimensional shape of an eardrum as disclosed in US2018168440A1. Herein, an otoscope is disclosed configured to project temporal sequences of phase-shifted fringe patterns onto an eardrum. A camera in the otoscope captures the reflected images and a computer reconstructs thereupon a three-dimensional map of the eardrum. The doctor then can rely on the three-dimensional map to make a diagnosis.

A drawback of using temporal sequences is that, firstly, the camera needs to be synchronized with the projector to relate the proper pattern to a proper captured reflection. Secondly, the phases of the sequences need to be shifted adequately to effectively reconstruct the three-dimensional map. This means that the phases need to precisely be shifted in time and space. This imposes severe constraints on the synchronization process.

Furthermore, the procedure of projecting patterns onto an eardrum and capturing the reflected images are performed in vivo. Because of this, the sequences may undesirably be affected by, for example, any motion of the patient, a movement of the otoscope in the ear canal because of a reaction of the patient, and/or swallowing movements of the patient. Especially when a child's eardrum is examined, these movements are not negligible and influence the outcome.

Finally, the procedure needs to be performed fast enough to limit any nuisance to the patient, to efficiently come to a proper diagnosis by the doctor, and to be able to diagnose a sufficiently high number of patients by a medical hospital such that costs related thereto are kept within limits.

It is therefore an object of the present invention to alleviate the above drawbacks and to provide an improved solution for reconstructing a three-dimensional map of an eardrum of a patient.

### Summary

This object is achieved, according to a first example aspect of the present disclosure, by a computer-implemented method for obtaining a three-dimensional map of an eardrum comprising the steps of:
- obtaining a reflection of a structured illumination pattern projected onto the eardrum; and
- constructing by a trained deep learning model the three-dimensional map based on the reflection.

A structured illumination pattern is, for example, a sinusoidal fringe or grid pattern wherefrom the different characteristics, such as the used frequencies, intensities for different zones, and the composition thereof are known. The structured illumination pattern may further comprise a pattern of structured dots, a pattern of structured ellipses, colour maps, or any other structured pattern suitable to derive information of a reflection thereof. It should further be understood that the structured illumination pattern originates from a source which is suitable to produce pattern for projecting it in vivo on an eardrum, and from which the characteristics are monitored, and adapted by an accuracy being suitable for performing a diagnosis of an eardrum.

The structured illumination pattern is projected onto an eardrum of a patient and subsequently reflected by the eardrum. Since an eardrum is partly translucent, a part of the projected illumination pattern is directly reflected, while a part is passed through the eardrum and reflected by a surface from the middle ear behind the eardrum. This latter reflection will then pass through the eardrum or is again reflected. The reflection obtained of the structured illumination pattern projected onto the eardrum thus comprises different components, such as the direct reflection, the partly indirect reflection from a surface of the middle ear behind the eardrum, and possibly a refraction of the indirect reflection, whereby the refraction is caused by the partly translucent eardrum. The different components are related to the characteristics of the eardrum, such as the shape, but also to the transparency and index of refraction. The reflection thus comprises an aggregation of different aspects which may mutually interact with each other.

Subsequently, the obtained reflection is used as an input for a trained deep learning model. The deep learning model is trained to construct three-dimensional maps of eardrums. Thus, by setting the reflection as an input for the trained deep learning model, the model constructs based on the reflection the three-dimensional map of the eardrum.

Different advantages are identified. Firstly, the procedure for constructing a three-dimensional eardrum is shortened to a number of controllable steps. Since only one reflection is sufficient to construct the three-dimensional map, there is no need for projecting sequences of structured illumination patterns, and consequently no need for synchronizing the projections and reflections. Secondly, the procedure is performed in an efficient manner such that no unnecessary burden is requested from the patient, such as keeping himself motionless during a relatively long period of time. Furthermore, there is no need to dye the eardrum, thereby reducing the time needed to perform the diagnosis. Finally, since a three-dimensional map is obtained, the doctor performs a diagnosis by the real shape of the eardrum instead of a two-dimensional image on a lens. This way, the accuracy of the diagnosis is increased.

Thus, it is an advantage that in contrast of using a set of sequences, and thus obtaining a three-dimensional map through a multi-shot procedure, the three-dimensional map is constructed by a single-shot whereby no phase-shift is required.

The constructed three-dimensional eardrum can be visualized by any means suitable for representing three-dimensional objects or images. For example, a two-dimensional screen can be used whereby the three-dimensional map can be rotated in a number of angles such that different perspectives are presented. Through the computer-implemented method, the constructed three-dimensional map per patient can also be stored such that a doctor can perform a follow-up in an efficient and correct manner at a later time.

According to an embodiment, the structured illumination pattern comprises a structured light pattern.

The structured light pattern comprises, for example, a grid pattern with a sinusoidal gray transition, or another colour of the visible light band. This way, the pattern is produced by using a number of elementary components, such as light-emitting diodes as a source for producing the illumination and a static pattern whereupon the illumination is projected.

According to an embodiment, the reflection comprises a deformed grid pattern.

In other words, the reflection is a deformed illumination pattern, whereby the deformation is caused by the different components as already highlighted. The deformed illumination pattern is thus representative for the shape of the eardrum, but also for the other mentioned characteristics. The deformed illumination pattern is then used as an input for the trained deep learning model by which the three-dimensional map, and thus the shape of the eardrum is constructed.

According to an embodiment, the reflection comprises a two-dimensional representation.

The obtained reflection can be represented by an ordered set of arrays, such as a matrix, and/or may be represented by a two-dimensional image file format. In other words, the reflection comprises data which can be represented by a table comprising values or numbers describing the reflection. The two-dimensional representation is then used as an input for the trained deep learning model.

According to an embodiment, the deep learning model is a convolutional neural network.

The trained deep learning model may be a trained convolutional neural network. By using a convolutional neural network, the non-linearity of the different characteristics of the eardrum and the middle ear influencing the reflection can be taken into account. The convolutional neural network is then configured to receive the reflection, in case represented as a two-dimensional array, and to output the three-dimensional map of the eardrum.

According to a second aspect, the disclosure relates to a data processing circuitry comprising means for carrying out the method according to the first aspect.

In other words, the steps of obtaining a reflection and constructing based thereon the three-dimensional map can be carried out by the data processing circuitry. The trained deep learning model, or in case the convolutional neural network, can be integrated in the circuitry. Alternatively, the means of the circuitry are configured to communicate with the trained deep learning model, or in case the trained convolutional neural network, such that the reflection is exchanged, and the constructed three-dimensional map is received in reply.

According to an embodiment, the circuitry comprises one of the group of a field-programmable gate array, FPGA, a graphics processing unit, GPU, a neural processing unit, NPU, and/or an artificial intelligence, AI, accelerator.

The circuitry may comprise a variety of microprocessors and/or units, like a FPGA, a GPU, an NPU, and/or an AI accelerator. These processors or units may further be combined by a parallel hardware architecture. The trained deep learning model is then integrated such that it is ready for inferences through parallel computing. This way performing inferences on new data by the trained deep learning model can be made at a real-time speed.

According to a third aspect, the disclosure relates to an otoscope comprising:
- a projector for projecting a structured illumination pattern onto an eardrum; and
- a camera for capturing a reflection of the structured illumination pattern; and
- means for exchanging the reflection with the circuitry according to the second aspect for constructing a three-dimensional map of the eardrum.

Thus, the otoscope comprises a projector, a camera, and means for exchanging data with a circuitry. The projector is configured to project a structured illumination pattern onto an eardrum. It comprises an illumination source, such as light-emitting diodes, and means to generate the pattern by interfering with the illumination source. This means is further identified as a fringe pattern.

The fringe pattern may be a static pattern printed, etched or lasered onto a piece of glass or plastic. The illumination will then pass through this component resulting into the structured illumination pattern. The means may also comprise a liquid crystal display sensor whereupon the illumination is projected, and subsequently reflected onto the eardrum. Through the orientation of the liquid crystals, the fringe pattern is adapted or modified. Alternatively, multiple small mirrors which can switch between a reflection and a non-reflection modus can be used to construct the fringe pattern.

Since no multi-shot set up is needed, nor any synchronization process needed to be implemented, nor a phase-shift, the camera or imaging device only needs to fulfil a few given specifications. These specifications are, among others, size, frame rate, and the delivered format. Given these specifications high quality cameras used in smartphones are suitable hereto. Furthermore, because of the required hardware setup, there is no need for flash memory to store pattern sequences. Finally, these requirements lead to more lightweight equipment, which is an advantage for a person, for example a doctor, who needs to handle the otoscope.

The means are configured to exchange the reflection captured by the camera with the circuitry for obtaining the reflection and constructing based thereupon the three-dimensional map. The circuitry is, in this embodiment, external from the otoscope. This way, the power and/or processing requirements of the otoscope are limited to the projecting and capturing steps. This way, the otoscope can be used for a successive number of diagnosis, while the processing of the reflection into the three-dimensional map is performed externally, with preferably increased processing capabilities.

According to an embodiment, the otoscope comprises the circuitry according to the second aspect.

The otoscope may also comprise the circuitry itself such that the otoscope is able to construct the three-dimensional map. This way, the otoscope can be used outside a diagnosis center, for example, when a doctor visits a patient at home.

According to an embodiment, the otoscope further comprises a display screen for displaying the three-dimensional map of the eardrum.

Optionally, the otoscope may also comprise a display screen whereupon the constructed three-dimensional map can be represented. This way, the doctor immediately sees the three-dimensional map such that he can promptly perform his diagnosis.

According to a fourth aspect, the disclosure relates to a deep learning model trained to construct a three-dimensional map of an eardrum according to the method of the first aspect.

The deep learning model is, for example, trained offline with a number of different structured illumination patterns and the model is trained such that is able to recognize or reconstruct related three-dimensional height maps thereof.

According to an embodiment, the neural network is trained by a training dataset comprising a plurality of height maps and corresponding deformed grid patterns.

The dataset comprising the plurality of height maps and corresponding deformed grid patterns may be constructed using simulation software. The dataset is then generated into known three-dimensional shapes and corresponding deformed grid patterns. This way, the angle of incidence of the projected light patterns may be selected to mimic an actual angle of incidence when integrated into the circuitry. This dataset is then used to train the deep learning model, or in case the convolutional neural network.

According to an embodiment the plurality of height maps comprises height maps of ex vivo eardrums.

In other words, eardrums from cadavers can be mapped and single-shot reflections can be measured as well. Based hereon, a dataset is generated which closely approximates real-life situations.

### Brief Description of the Drawings

Some example embodiments will now be described with reference to the accompanying drawings.
Fig. 1 illustrates the examination of a patient's ear by a doctor by the use of an otoscope according to an example embodiment of the invention; and
Fig. 2 illustrates a cross-section of an ear; and
Fig. 3 illustrates a cross-section of an ear corresponding to the illustration of Fig. 2 comprising an eardrum whereupon a light beam is projected; and
Fig. 4 shows an otoscope according to an example embodiment of the invention projecting a light beam on an eardrum; and
Fig. 5 shows an example embodiment of structured light patterns and related three-dimensional maps; and
Fig. 6 shows an example embodiment of a circuitry comprising a deep learning model for constructing a three-dimensional map; and
Fig. 7 shows steps performed to construct a three-dimensional map of an eardrum based on a reflection; and
Fig. 8 shows an example embodiment of a suitable computing system for performing one or several steps in embodiments of the invention; and
Fig. 9 illustrates a cloud-interface to execute one or more embodiments of constructing a three-dimensional map of an eardrum in the cloud.

### Detailed Description of Embodiment(s)

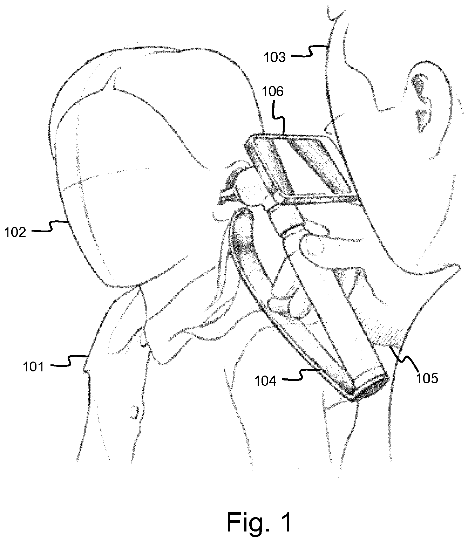

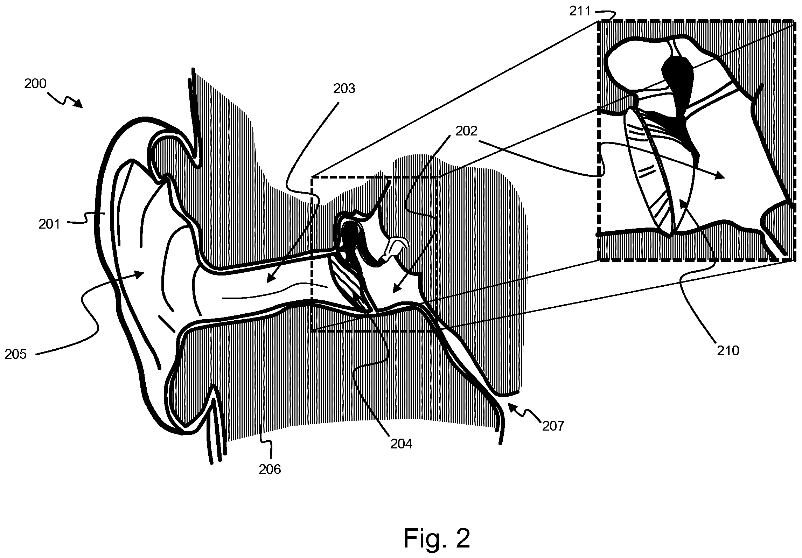

Fig. 1 illustrates a patient 101 from which his or her ear is examined by a doctor 103. The examination is performed by an otoscope 104 allowing the doctor 103 to look into the patient's 101 ear. The examining is performed by emitting a light-beam originating from the otoscope 104 into the ear canal. In Fig. 2 an ear 205 is illustrated comprising an auricle 201, an ear canal 203, and an eardrum 204. The illustrated cross-section 200 of the ear further illustrated the middle ear 202 and the eustachian tube 207. The shaded surface 206 illustrates the part of the patient's 101 head 102 surrounding the ear 201 and different parts therefore. It should be further understood that the cross section 200 represents an ear in a condition appropriate to be examined by the doctor 103, even if the patient 101 would suffer from diseases and/or discomforts.

In Fig. 2 a detailed view 211 of a part of the illustrated cross-section 200 is further represented. The detailed view 211 comprises the middle ear 202 and the eardrum 204, 210. From the detailed view 211 of the eardrum 210 it should be clear that the eardrum 210 comprises a three-dimensional surface.

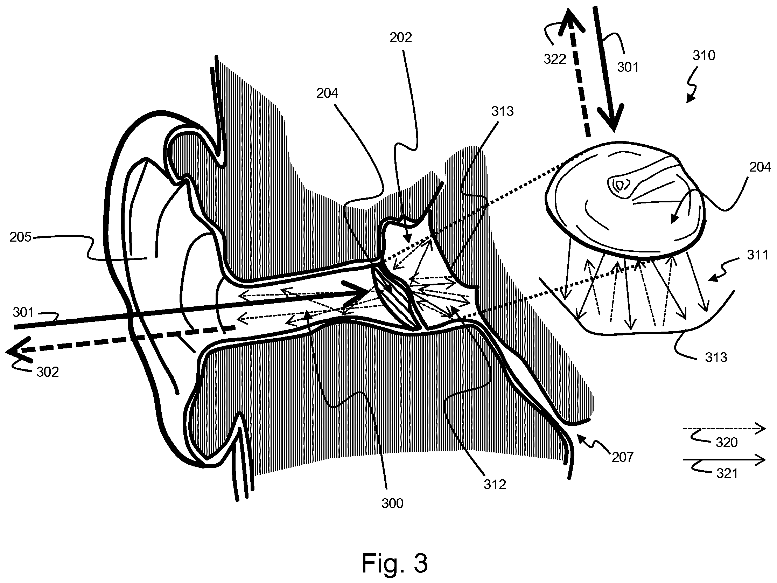

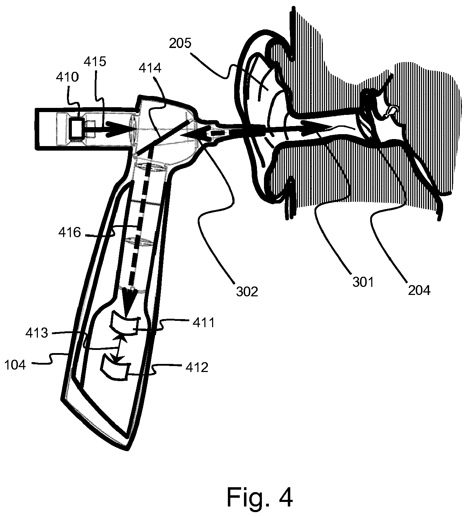

When the doctor 103 examines the patient's 101 ear 205, and in particular the internal part thereof, a light-beam is projected on the eardrum 204. In Fig. 4 the otoscope 104 is further illustrated together with the ear 205. From the otoscope 104 a light-beam, represented by arrow 301, is projected on the eardrum 204. The light-beam 301 is further illustrated in Fig. 3 which illustrated the ear 205, as already discussed. The arrow 301 in Fig. 3 likewise represents the light-beam originating from the otoscope 104.

When a patient 101 consults a doctor 103, for example when he or she suffers from ear pain, the suffering may indicate an infection and/or injury in the middle ear 202. Yet, since the eardrum 204 forms a barrier between the ear canal 203 and the middle ear 202, the doctor 103 is unable to directly examine the middle ear 202. Hence, the doctor 103 examines the eardrum 204 and based thereon a diagnosis can be made.

To increase the accuracy of diagnoses, the doctor 103 obtains a three-dimensional view of the eardrum 204, 210. In other words, the otoscope 104 is configured to construct a three-dimensional map of the eardrum 204, 210.

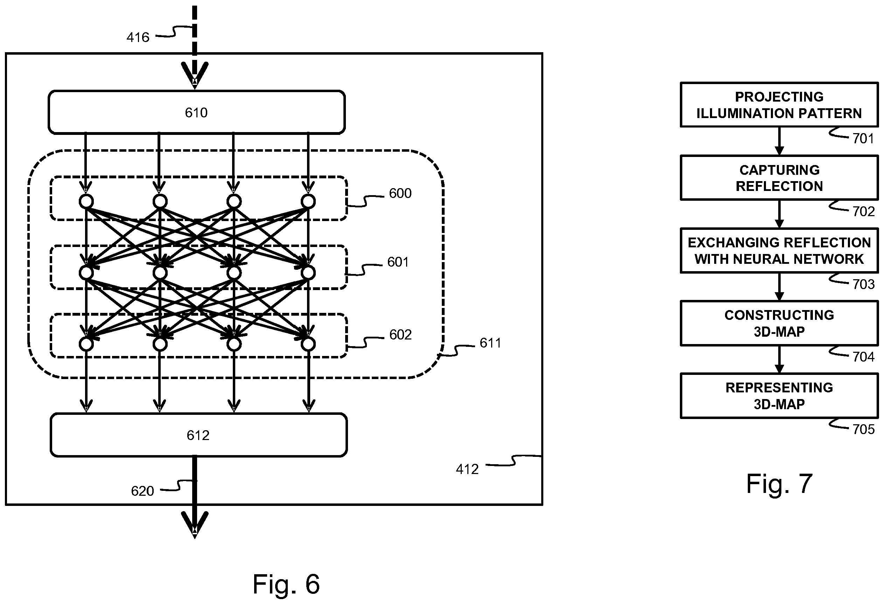

The steps performed to obtain such a three-dimensional map will further be illustrated with reference to Fig. 7.

Firstly, the light-beam 301 is projected 701 on the eardrum 204, in particular on the surface of the eardrum 204 facing the ear canal 203, whereby the eardrum 204 is partly translucent. The degree of transparency may vary and can, approximately, be estimated to be around seventy-five percent, which means that seventy-five percent of emitted light-beams is passed through, while twenty-five percent is immediately reflected back. It should, however, further be understood that the degree of transparency may vary and that the specified number serves as an example and thus not limit the invention.

Thus, a part of the projected 701 light-beam 301 passes through the surface of the eardrum 204, 211. This is further illustrated by the arrows 321 represented in the middle ear 312 of the illustration of Fig. 3. The transmitted light-beams 321 on their behalf are reflected by the walls 313 of the middle ear 202. This is further illustrated by detailed view 310, which represents the eardrum 204 and further represents light-beams 311 between the eardrum and the middle ear's 202 walls 313. The light-beams reflected by the middle ear's 202 walls 313 are further nominated as indirectly reflected light-beams.

Subsequently, the transmitted light-beams 321 reflected by the walls 313 result in light-beams 320. These light-beams 320, or a part thereof, passes through the eardrum 204 from the middle ear 202 to the ear canal 203. The indirectly reflected light-beams may further be refracted by the eardrum 204 when transmitted towards the ear canal 203, depending on the index of refraction of the eardrum 204. These refracted and transmitted light-beams are result in a first resultant 322.

Additionally and/or simultaneously, a part of the light-beam 301 is immediately reflected by the eardrum 204. As a result, in the ear canal, a complex combination of directly and indirectly reflected light-beams are present. This is further illustrated by reference 300. The final resultant of this complex combination of light-beams, thus comprising the first resultant 322 and the directly reflected light-beams, is represented by arrow 302.

Since during examination of the patient 101, the patient 101 may move his head 102, potentially combined with movements of the doctor's 103 hand 105, the otoscope 104 is configured to construct in a fast manner a three-dimensional map of the eardrum 204 such that any nuisance to the patient 101 is limited. This is achieved by projecting a single-shot light-beam 301 onto the eardrum 204 and by capturing the projected resultant 302 to derive therefrom the three-dimensional map of the eardrum 204.

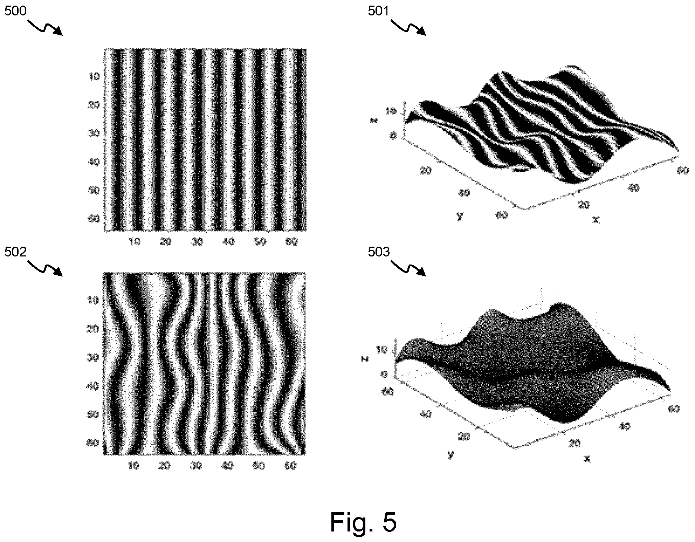

Thereto, the otoscope 104 comprises a projector 410 configured to produce the light-beam 301. More in particular the projector 410 is configured to produce a structured illumination pattern. An example of a structured illumination pattern is illustrated in Fig. 5. Illumination pattern 500 illustrates a grid pattern with ten sin wave periods, but it should be further understood that the invention is not limited to an illumination pattern 500 comprising these parameters. It should be further understood that the projector 410 is either configured to produce directly the structured illumination pattern, or indirectly by, for example, the use of one or more filters.

The light-beam comprising the structured illumination pattern 500 is further illustrated by arrow 415. The otoscope 104 may further comprise a semi-transparent mirror 414 configured to focus the beam 415 such that it is optimally projected on the eardrum 204 by beam 301.

It should be further understood that the otoscope 104 comprises a plurality of components besides the projector 410 and the semi-transparent mirror 414 allowing the doctor 103 to optimally project a structured illumination pattern 500 onto the eardrum 204. These components may, but not limited thereto, be collimator lenses, a projection lens, replaceable specula, and/or an imaging lens. It should be further understood that the position of the projector 410 in the otoscope 104 and the semi-transparent mirror 414, and other components like a camera 411, and a circuitry 412 are an illustrative embodiment of the otoscope 104 but may be positioned differently in other example embodiments.

Thus, the light-beam 301 comprising the structured illumination pattern 500 is projected 701 onto the eardrum 204. A result of the projected pattern 500 may be illustrated by the three-dimensional surface 501 comprising fringes or distorted patterns. Thus, this corresponds to the reflected light-beam 302 comprising the complex combination of already discussed directly and indirectly reflected parts of the structured illumination pattern 500.

The complex combination of distorted or deformed arrangement of fringes of the pattern may further be presented as a two-dimensional image. This two-dimensional image is illustrated by the two-dimensional distorted illumination pattern 502. Further, this two-dimensional illumination pattern 502 is captured 702 by the otoscope 104. The capturing 702 may, for example, be performed by deviating the reflected light-beam 302 to a camera 411 by the semi-transparent mirror 414 within the otoscope 104. This deviated light-beam is represented by the beam 416. The beam 416 thus comprises the information comprised by the reflected beam 302.

The beam 416, thus the two-dimensional distorted illumination pattern 502, captured 702 by the camera 411 serves as an input for a circuitry illustrated by Fig. 6. Fig. 6 illustrates a circuitry 412 comprising a deep learning model 611, such as, for example, a convolutional neural network.

The illustrated deep learning model 611 comprises three layers 600-602, but it should be further understood that the illustrated model 611 may comprise a different number of layers and/or a different number of connected nodes. The illustrated deep learning model 611 thus serves as an illustration of the invention and does not limit the invention to the use of the illustrated deep learning model 611.

The circuitry 412 may be incorporated in the otoscope 104, but, according to other illustrative embodiments, the otoscope 104 may comprise an interface configured to communicate with the circuitry 412, whereby the circuitry 412 may be located outside the otoscope 104. This exchange may be performed wired or wirelessly.

In the illustrative example of Fig. 4, the captured 702 reflection 502 is exchanged thus 413, 703 with the circuitry 412, wherein the circuitry 412 is incorporated in the otoscope 104. This captured 702 reflection 502 is illustrated as an input 416 for the circuitry 412 in Fig. 6. Alternatively, the camera 411 may be configured to receive the captured 702 reflection and to transform it into a data format suitable for the circuitry 412 for further processing. The captured 702 reflection 416 may by the circuity 412 itself be transformed such that it becomes suitable as an input for the deep learning model 611. The receive unit 610 thus illustrates a unit configured to receive a data set representing the reflection 502 and further configured to transform the data set suitable for inputting it to the deep learning model 611.

Further, the deep learning model 611 is configured and trained to construct 704 a three-dimensional map of the eardrum 204 based on the received reflection. The deep learning model 611 will thus transform the two-dimensional reflection 502 into a three-dimensional map 503.

Thus, in step 703 the reflection 502 is exchanged with the deep learning model 611, which in case may comprise a convolutional neural network. The trained deep learning model 611 constructs 704 based on the reflection 502 the three-dimensional map 503 of the eardrum 204. The constructed 704 three-dimensional map 503 may, for example, be further processed by unit 612 such that is becomes suitable for representing 705 it on a screen 106. Finally, the three-dimensional map 503, either an output of unit 612 or directly constructed 704 by the deep learning model 611 is forwarded such that the doctor 103 is able to make a diagnosis based thereon. This constructed 704 three-dimensional map 503 is represented by output 620.

The constructed 704 three-dimensional map 503, 620 may, for example, be represented 705 on a screen which may be incorporated into the otoscope 104, as illustrated by screen 106 in Fig. 1. Alternatively or additionally, the constructed 704 three-dimensional map 620 of the eardrum 204 may be represented on another screen configured to communicate with the circuitry 412.

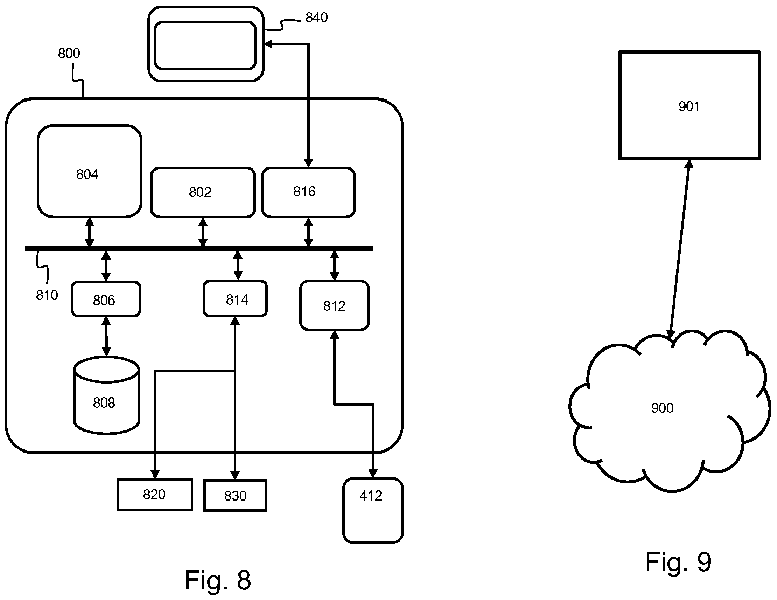

Fig. 8 shows a suitable computing system 800 enabling to implement embodiments of the method for constructing 704 a three-dimensional map 503 of an eardrum 204 based on a captured 702 of deformed illumination pattern 502. Computing system 800 may in general be formed as a suitable general-purpose computer and comprise a bus 810, a processor 802, a local memory 804, one or more optional input interfaces 814, one or more interface 806, and one or more storage elements 808. Bus 810 may comprise one or more conductors that permit communication among the components of the computing system 800. Processor 802 may include any type of conventional processor or microprocessor that interprets and executes programming instructions. Local memory 804 may include a random-access memory (RAM) or another type of dynamic storage device that stores information and instructions for execution by processor 802 and/or a read only memory (ROM) or another type of static storage device that stores static information and instructions for use by processor 802. Input interface 814 may comprise one or more conventional mechanisms that permit an operator or user, such as the doctor 103, to input information to the computing device 800, such as a keyboard 820, a mouse 830, a pen, voice recognition and/or biometric mechanisms, a camera, etc. Output interface 816 may comprise one or more conventional mechanisms that output information to the operator or user, such as a display 840 like display 106, etc. Communication interface 812 may comprise any transceiver-like mechanism such as for example one or more Ethernet interfaces that enables computing system 800 to communicate with other devices and/or systems, for example with circuitry 412. The communication interface 812 of computing system 800 may be connected to such another computing system by means of a local area network (LAN) or a wide area network (WAN) such as for example the internet. Storage element interface 806 may comprise a storage interface such as for example a Serial Advanced Technology Attachment (SATA) interface or a Small Computer System Interface (SCSI) for connecting bus 510 to one or more storage elements 808, such as one or more local disks, for example SATA disk drives, and control the reading and writing of data to and/or from these storage elements 808. Although the storage element(s) 808 above is/are described as a local disk, in general any other suitable computer-readable media such as a removable magnetic disk, optical storage media such as a CD or DVD, -ROM disk, solid state drives, flash memory cards, ... could be used. Computing system 800 could thus correspond to the controller circuitry 412 in the embodiments illustrated by Fig. 4 or Fig. 6.

The steps according to the above embodiments may be performed on computing system 800. Computing system may interact directly with a user such as the doctor 103, e.g. through the interfaces 820, 830 and represent the results of the steps according to the above embodiments on a display 840, such as display 106, or print them on a printer 850. Alternatively, as illustrated in Fig. 9, the steps may be performed remote from a user on a remote computing system 900, e.g., on a cloud computing system. Interaction with a user may then be done by a connection between the remote computing system 900 and a client computing system 901, e.g. over an Internet connection. Also, the client computing system 901 may be implemented as computing system 800. Communication is then performed over a wired or wireless networking interface 812.

As used in this application, the term "circuitry" may refer to one or more or all of the following:
(a) hardware-only circuit implementations such as implementations in only analogue and/or digital circuitry and
(b) combinations of hardware circuits and software, such as (as applicable):
   (i) a combination of analogue and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions) and
(c) hardware circuit(s) and/or processor(s), such as microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example and if applicable to the particular claim element, a baseband integrated circuit or processor integrated circuit for a mobile device or a similar integrated circuit in a server, a cellular network device, or other computing or network device.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the scope of the claims are therefore intended to be embraced therein.

It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A computer-implemented method for obtaining a three-dimensional map (503) of an eardrum (204) comprising the steps of:
- obtaining (702) a reflection (302, 502) of a structured illumination pattern (301, 500) projected (701) onto the eardrum (204); and
- constructing (704) by a trained deep learning model (611) the three-dimensional map (503) based on the reflection (302, 502).

2. The computer-implemented method according to claim 1, wherein the structured illumination pattern (301, 500) comprises a structured light pattern.

3. The computer-implemented method according to one of the preceding claims, wherein the reflection (302, 502) comprises a deformed grid pattern.

4. The computer-implemented method according to one of the preceding claims, wherein the reflection (302, 502) comprises a two-dimensional representation.

5. The computer-implemented method according to one of the preceding claims, wherein the deep learning model (611) is a convolutional neural network.

6. A data processing circuitry (412) comprising means for carrying out the method according to one of the preceding claims.

7. The data processing circuitry (412) according to claim 6 further comprising one of the group of a field-programmable gate array, FPGA, a graphics processing unit, GPU, a neural processing unit, NPU, and/or an artificial intelligence, Al, accelerator.

8. An otoscope (104) comprising:
- a projector (410) for projecting (701) a structured illumination pattern (301, 500) onto an eardrum (204); and
- a camera (411) for capturing (702) a reflection (302,502) of the structured illumination pattern (301, 500); and
- means for exchanging (703) the reflection (302, 502) with the circuitry (412) according to claim 6 or 7 for constructing (704) a three-dimensional map (503) of the eardrum (204).

9. The otoscope (104) according to claim 8 further comprising the circuitry (412).

10. The otoscope (104) according to one of the claims 8 or 9 further comprising a display (106) screen for displaying the three-dimensional map (503) of the eardrum (204).

11. A deep learning model (611) trained to construct (704) a three-dimensional map (503) of an eardrum (204) according to the method of one of the claims 1 to 5.

12. The deep learning model (611) according to claim 11 wherein the deep learning model (611) is trained by a training dataset comprising a plurality of height maps and corresponding deformed grid patterns.

13. The deep learning model (611) according to claim 12, wherein the plurality of height maps comprises height maps of ex vivo eardrums.

14. A computer program comprising instructions which, when the program is executed by a computer, cause the computer to carry out the method according to one of the claims 1 to 5.

15. A computer-readable data carrier having stored thereon the computer program of claim 14.
